# EUROPEAN PATENT SPECIFICATION

(11) **EP 3 246 307 B1**
(45) Date of publication and mention of the grant of the patent: **27.11.2019**
(21) Application number: 16737381.0
(22) Date of filing: 13.01.2016
(51) Int. Cl.: C07C 201/12, C07C 205/42

(54) **METHOD FOR PRODUCING DIAMINE PRECURSOR COMPOUND**
VERFAHREN ZUR HERSTELLUNG EINER DIAMINVORLÄUFERVERBINDUNG
PROCÉDÉ DE PRODUCTION D'UN COMPOSÉ PRÉCURSEUR DE DIAMINE

(30) Priority: 13.01.2015 JP 2015004366
(43) Date of publication of application: 22.11.2017
(73) Proprietor: Nissan Chemical Corporation, Tokyo (JP)
(72) Inventor: NAGAO, Masato, Funabashi-shi Chiba 274-0052 (JP)
(74) Representative: Blodig, Wolfgang
(86) International application number: PCT/JP2016/050851
(87) International publication number: WO 2016/114310

(56) References cited:
- WO-A1-2014/208609
- JP-A- S6 025 955
- JP-A- 2005 112 764
- JP-A- 2007 204 438
- JP-A- 2008 133 262
- JP-A- 2014 006 442
- ALLEN R. BANKS ET AL.: 'A Convenient Synthesis of Methacrylates' J. ORG. CHEM. vol. 42, no. 24, 01 January 1977, pages 3965 - 3966, XP055466949

## Description

### TECHNICAL FIELD

The present invention relates to a novel method for efficiently producing a highly pure dinitro compound which is a precursor of a specific diamine compound as a starting material for producing a polyimide to be used as a liquid crystal aligning agent.

### BACKGROUND ART

The diamine compound is widely used as one type of a monomer which is a starting material for a polymer such as a polyamide, a polyamic acid, a polyimide, a polyamic acid ester, a polyamide imide and a polyether imide. Such polymers are likely to have excellent mechanical strength and heat resistance and thereby used in various applications such as members for automobile and home appliances.

Further, a polyimide, and a polyamic acid and a polyamic acid ester which are precursors of a polyimide are commonly used as a liquid crystal alignment film which is one of constituent members of a liquid crystal display device.

The liquid crystal alignment film is produced by the method of rubbing a polymer coating film formed on an electrode substrate by a cloth made of e.g. rayon under pressure, so called rubbing treatment.

Further, a method has been known that by rubbing a polymer coating film formed on a substrate toward plural directions and tilting liquid crystal molecules along the rubbing directions, domains toward plural directions are formed, whereby alignment division required for wide viewing angle can be realized (Patent Document 1).

Further, a method has been known that a polyimide comprising a specific diamine compound is used as a liquid crystal alignment film, whereby a pretilt angle can be controlled (Patent Document 2).

As another method for forming a liquid crystal alignment film, there is a method, so called photo-alignment in which a polymer coating film is irradiated with polarized or non-polarized ultraviolet ray (Patent Document 3).

It has been known that in this method, by using a polyimide comprising a specific diamine compound as a liquid crystal alignment film, a desired pretilt angle can be easily formed (Patent Document 4).

Further, there is another method to align liquid crystal, which includes in the production steps, a step of irradiating liquid crystal molecules with ultraviolet ray while applying voltage. In such a method, it is common to preliminary add a photo-polymerizable compound in a liquid crystal composition (Patent Document 5).

Further, similarly to the above-mentioned photo-alignment, a method has been known that photoreactive groups are incorporated in a polymer of a liquid crystal alignment film (Non-Patent Document 1). Further, a method has been known that a photo-polymerizable compound is not added (Patent Document 6).

A diamine compound having 2-(2,4-diaminophenyl)ethylester structure is commonly included in the specific diamine compounds mentioned in Patent Document 2, Patent Document 4 and Patent Document 6. Such a diamine compound is synthesized by reducing a compound having 2-(2,4-dinitrophenyl)ethylester Production of the latter is known from TW201348299 by reacting an acyl chloride of formula 2 and an alcohol of formula 1, as shown below, in the presence of triethylamine as a base.

### PRIOR ART DOCUMENTS

### PATENT DOCUMENTS

Patent Document 1: JP-A-2005-316363
Patent Document 2: WO2013/137333
Patent Document 3: JP-A-2003-114437
Patent Document 4: JP2013/519744
Patent Document 5: JP-A-2003-307720
Patent Document 6: WO2013/099804

### NON-PATENT DOCUMENT

Non-Patent Document 1: SID 10 DIGEST, p. 595

### DISCLOSURE OF INVENTION

### TECHNICAL PROBLEM

It is an object of the present invention to provide a novel method for efficiently producing a highly pure 2-(2,4-dinitrophenyl)ethylester derivative.

### SOLUTION TO PROBLEM

The present inventors have extensively studied in order to accomplish the above object, and as a result, they have accomplished the present invention. That is, the present invention is described below.
1. A method for producing a diamine precursor compound represented by the formula 3, which comprises reacting, in accordance with the following reaction formula (1), a dinitro compound represented by the formula 1 and a compound represented by the formula 2 in the absence of a base, while discharging generated hydrogen chloride in the form of gas to the outside of the reaction system: 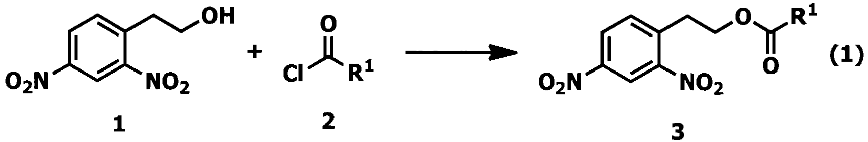 where R¹ is a monovalent organic group.
2. The production method according to the above 1, wherein a reaction solvent is further used.
3. The production method according to the above 1, wherein an aromatic hydrocarbon is used as a reaction solvent.
4. The production method according to the above 1, wherein toluene is used as a reaction solvent.
5. The production method according to any one of the above 1 to 4, wherein the amount of a reaction solvent to be used is from 1 to 50 times by mass per the dinitro compound represented by the formula 1.
6. The production method according to any one of the above 1 to 5, wherein the reaction is carried out at a temperature of from 50 to 120°C.
7. The production method according to any one of the above 1 to 6, wherein the reaction is carried out under reduced pressure of from 100 to 700 mmHg by the absolute pressure.
8. The production method according to any one of the above 1 to 7, wherein the amount of the compound represented by the formula 2 to be used is from 0.5 to 3.0 times by mol per the dinitro compound represented by the formula 1.
9. The production method according to any one of the above 1 to 8, wherein the compound represented by the formula 2 is a compound represented by the formula 4.

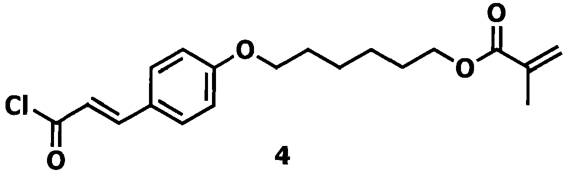

### ADVANTAGEOUS EFFECTS OF INVENTION

According to the present invention, it is possible to efficiently produce from 2-(2,4-dinitrophenyl)ethanol and a carboxylic acid chloride, a highly pure dinitro compound which is a precursor compound of a diamine compound which is a starting material of a polyamide acid and/or a polyimide to be used as a liquid crystal alignment agent.

### DESCRIPTION OF EMBODIMENTS

The production method of the present invention is in accordance with the following reaction formula (1). That is, the present invention is a method for producing a diamine precursor compound represented by the formula 3, which comprises reacting a dinitro compound represented by the formula 1 and a compound represented by the formula 2 (wherein R¹ is a monovalent organic group), while discharging generated hydrogen chloride in the form of gas to the outside of the reaction system.

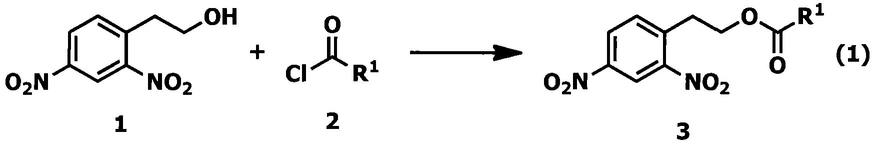

That is, a dinitro phenethyl alcohol represented by the formula 1 and a carboxylic acid chloride represented by the formula 2 are reacted to obtain a dinitro compound represented by the formula 3.

The amount of an acid chloride represented by the formula 2 to be used is preferably from 0.5 to 3.0 times by mol, more preferably from 0.9 to 1.5 times by mol, per the dinitro compound represented by the formula 1.

In the reaction represented by the formula (1), a reaction solvent is preferably used. As the reaction solvent, any organic solvent may be used, so long as the reaction solvent will not react with the acid chloride represented by the formula 2 and can be uniformly mixed with starting materials. Particularly, an aromatic hydrocarbon solvent such as benzene, toluene or xylene is preferred, and toluene is particularly preferred.

The amount of the reaction solvent to be used is preferably from 1 to 50 times by mass, more preferably from 3 to 15 times by mass, per the dinitro compound represented by the formula 1.

The reaction temperature is from room temperature to the boiling point of the solvent. However, the reaction temperature is preferably from 50 to 120°C, particularly preferably from 60 to 120°C in order to efficiently remove by-produced hydrogen chloride and let the reaction proceed smoothly without a base.

The reaction time is preferably from 0.1 to 50 hours, particularly preferably from 0.5 to 30 hours.

After the reaction, the reaction solution is treated to obtain the desired product as described below. After the reaction, the reaction solution is concentrated under reduced pressure to obtain a reaction crude product. The reaction crude product is heated to from 30 to 77°C, more preferably from 40 to 70°C and dissolved in ethyl acetate, followed by cooling to room temperature. Then, the solution is washed with water, concentrated under reduced pressure and dried under reduced pressure (preferably from 0.1 to 200 mmHg by gauge pressure, and the temperature is preferably from 10 to 80°C, more preferably from 25 to 50°C) to obtain the desired compound.

The reaction may be carried out under normal pressure, reduced pressure or increased pressure and may be carried out by a batch process or a continuous process. The reaction may be carried out under normal pressure or reduced pressure, however, the reaction is preferably carried out under reduced pressure of from 100 to 700 mmHg by absolute pressure, more preferably from 200 to 600 mmHg, in order to efficiently remove generated hydrogen chloride.

It is known that if a base is added, reactions similar to the present reaction usually proceed smoothly. However, in the present invention, without adding a base, the reaction can smoothly proceed, and the desired dinitro compound can be obtained at a high yield, while suppressing by-production of 2,4-dinitrostyrene.

The dinitro compound represented by the formula 1 which is one of starting materials is a known compound, and WO2012/005266 discloses reducing 2,4-dinitrophenyl acetic acid with borane dimethyl sulfide complex to obtain the dinitro compound. Further, as 2,4-dinitrophenyl acetic acid, a commercially available one may be used (for example, manufactured by Tokyo Chemical Industry Co., Ltd.).

The acid chloride represented by the formula 2 which is the other starting material can be obtained by halogenating a known carboxylic acid with various halogenating agents. For example, an acid chloride is obtained at a high yield from a known carboxylic acid by using thionyl chloride or oxalyl chloride as the halogenating agent.

The amount of the halogenating agent to be used is preferably from 0.5 to 2.0 times by mol, particularly preferably from 1.0 to 1.5 times by mol, per the carboxylic acid. The reaction temperature is preferably from 0 to 80°C.

The dinitro compound thus obtained is subjected to a reduction reaction by a known method to form a diamine compound.

The obtained diamine compound is useful as a starting material for a polyimide (precursor) which is suitably used in various types of display devices such as TN liquid crystal display device, STN liquid crystal display device, TFT liquid crystal display device, OCB liquid crystal display device, in-plane-switching (IPS) liquid crystal display device and VA liquid crystal display device.

### EXAMPLES

Now, the present invention will be described in further detail with reference to Examples. Further, in Example, used analyzing devices and analyzing conditions are mentioned below.

### HPLC Analysis

Apparatus: LC-20A system (manufactured by Shimadzu Corporation)
Column: Inertsil ODS-3 (4.6 mm Φ × 250 mm, manufactured by GL Sciences Inc.)
Detector: UV detector (wavelength: 254 nm)
Eluting solution: Acetonitrile/0.1 wt% acetic acid aqueous solution (70/30, v/v)

### REFERENCE EXAMPLE 1

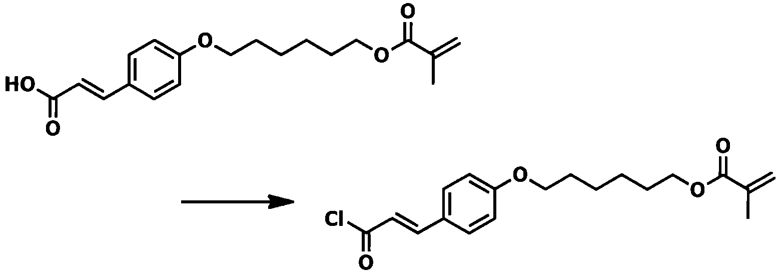

(E)-4-(6-(methacryloyloxy)hexyloxy) cinnamic acid (200 g, 602 mmol) and DMF (2 g) were added to toluene (1,000 g), and while heating at 70°C, thionyl chloride (89.5 g, 752 mmol) was dropwise added thereto over 1 hour. Then, after stirring for 1 hour at 70°C, the solvent was distilled off under reduced pressure to obtain (E)-4-(6-(methacryloyloxy)hexyloxy) cinnamoyl chloride (211 g).

### EXAMPLE 1

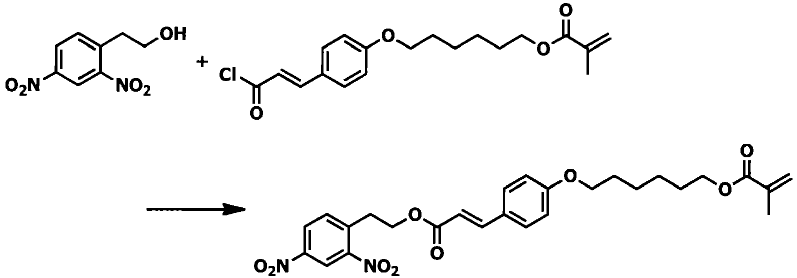

2-(2,4-dinitrophenyl)ethanol (128 g, 601 mmol) was added to toluene (422 g), and while heating at 90°C, a solution having (E)-4-(6-methacryloyloxy)hexyloxy) cinnamoyl chloride (211 g, 601 mmol) dissolved in toluene (422 g) was dropwise added thereto over 25 minutes. Then, after stirring at 90°C for 2 hours, the mixture was measured by HPLC, and it was confirmed that the area ratio of (E)-4-(6-methacryloyloxy)hexyloxy) cinnamoyl (2-(2,4-dinitrophenyl)ethyl)ester to 2,4-dinitrostyrene was 100:0.

Then, the reaction liquid was cooled, and water (422 g) and ethylacetate (1,477 g) were added, followed by stirring for 10 minutes. The reaction solution was allowed to stand, and then the water layer was removed. Then, a 5 wt% sodium hydrogen carbonate aqueous solution (422 g) was added to the organic layer, followed by stirring for 5 minutes. After being allowed to stand, the water layer was removed. Then, water (422 g) was added to the organic layer, followed by stirring for 5 minutes. After being allowed to stand, the water layer was removed. Then, the organic layer was distilled off under reduced pressure, and ethyl acetate (352 g) was added to the residue to dissolve the residue. Then, methanol (1,059 g) was dropwise added to the dissolved residue at 25°C over 30 minutes to precipitate crystal. Then, after cooling at 5°C, the precipitated crystal was filtered, followed by drying to obtain (E)-4-(6-methacryloyloxy)hexyloxy) cinnamic acid (2-(2,4-dinitrophenyl)ethyl)ester (outer appearance: yellow solid, obtained amount 281 g, yield: 89%, HPLC relative area percent: 99.1%).

### COMPARATIVE EXAMPLE 1:

2-(2,4-dinitrophenyl)ethanol (0.32 g, 1.5 mmol) and (E)-4-(6-(methacryloyloxy)hexyloxy) cinnamic acid (0.50 g, 1.5 mmol) were added to dichloromethane (4.0 g), and EDC-HCl (1-ethyl-3-(3-dimethylaminopropyl) carbodiimide hydrochloride) (0.72 g, 3.8 mmol) and DMAP (N,N-dimethyl-4-aminopyridine) (0.037 g, 0.30 mmol) were added thereto, followed by stirring at room temperature. After 20 hours, the mixture was measured by HPLC, and as a result, the area ratio of (E)-4-(6-methacryloyloxy)hexyloxy) cinnamic acid (2-(2,4-dinitrophenyl)ethyl)ester to 2,4-dinitrostyrene was 62:37.

### INDUSTRIAL APPLICABILITY

According to the production method of the present invention, a diamine compound which is useful as a starting material for a liquid crystal alignment agent can be simply and efficiently produced at a high purity from an inexpensive starting material, and a large industrial production can be carried out.

## Claims

1. A method for producing a diamine precursor compound represented by the formula 3, which comprises reacting, in accordance with the following reaction formula (1), a dinitro compound represented by the formula 1 and a compound represented by the formula 2 in the absence of a base, while discharging generated hydrogen chloride in the form of gas to the outside of the reaction system: 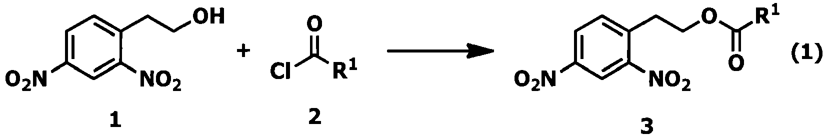 where R¹ is a monovalent organic group.

2. The production method according to Claim 1, wherein a reaction solvent is further used.

3. The production method according to Claim 1, wherein an aromatic hydrocarbon is used as a reaction solvent.

4. The production method according to Claim 1, wherein toluene is used as a reaction solvent.

5. The production method according to any one of Claims 1 to 4, wherein the amount of a reaction solvent to be used is from 1 to 50 times by mass per the dinitro compound represented by the formula 1.

6. The production method according to any one of Claims 1 to 5, wherein the reaction is carried out at a temperature of from 50 to 120°C.

7. The production method according to any one of Claims 1 to 6, wherein the reaction is carried out under reduced pressure of from 100 to 700 mmHg by the absolute pressure.

8. The production method according to any one of Claims 1 to 7, wherein the amount of the compound represented by the formula 2 to be used is from 0.5 to 3.0 times by mol per the dinitro compound represented by the formula 1.

9. The production method according to any one of Claims 1 to 8, wherein the compound represented by the formula 2 is a compound represented by the formula 4.

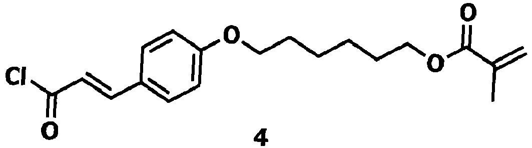

## Patentansprüche

1. Verfahren zur Herstellung einer Diamin-Vorläuferverbindung der Formel 3, welches das Umsetzen, in Übereinstimmung mit der folgenden Reaktionsformel (1), einer Dinitroverbindung der Formel 1 und einer Verbindung der Formel 2 in Abwesenheit einer Base umfasst, während erzeugter Chlorwasserstoff in Form eines Gases aus dem Reaktionssystem abgeführt wird: 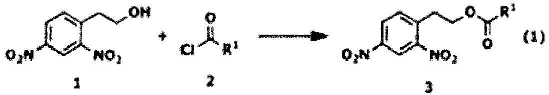 wobei R¹ eine einwertige organische Gruppe ist.

2. Herstellungsverfahren nach Anspruch 1, wobei ferner ein Reaktionslösungsmittel verwendet wird.

3. Herstellungsverfahren nach Anspruch 1, wobei ein aromatischer Kohlenwasserstoff als Reaktionslösungsmittel verwendet wird.

4. Herstellungsverfahren nach Anspruch 1, wobei Toluol als Reaktionslösungsmittel verwendet wird.

5. Herstellungsverfahren nach einem der Ansprüche 1 bis 4, wobei die Menge des Reaktionslösungsmittels, das verwendet werden soll, das von 1- bis 50-fache nach Masse der Dinitroverbindung der Formel 1 beträgt.

6. Herstellungsverfahren nach einem der Ansprüche 1 bis 5, wobei die Umsetzung bei einer Temperatur von 50 bis 120°C durchgeführt wird.

7. Herstellungsverfahren nach einem der Ansprüche 1 bis 6, wobei die Umsetzung unter vermindertem Druck von 100 bis 700 mmHg nach Absolutdruck durchgeführt wird.

8. Herstellungsverfahren nach einem der Ansprüche 1 bis 7, wobei die Menge der Verbindung der Formel 2, die verwendet werden soll, das von 0,5- bis 3,0-fache nach Mol der Dinitroverbindung der Formel 1 beträgt.

9. Herstellungsverfahren nach einem der Ansprüche 1 bis 8, wobei die Verbindung der Formel 2, eine Verbindung der Formel 4 ist

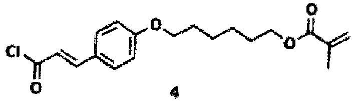

## Revendications

1. Procédé pour produire un composé précurseur de diamine représenté par la formule 3, qui comprend la réaction, conformément à la formule réactionnelle (1) qui suit, d'un composé dinitro représenté par la formule 1 et d'un composé représenté par la formule 2 en l'absence d'une base, cependant que le chlorure d'hydrogène généré est évacué sous forme de gaz vers l'extérieur du système réactionnel : 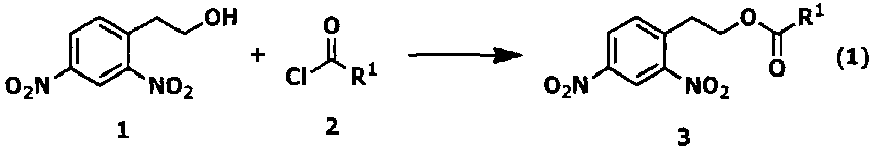 dans lequel R¹ est un groupe organique monovalent.

2. Procédé de production selon la revendication 1, dans lequel un solvant réactionnel est en outre utilisé.

3. Procédé de production selon la revendication 1, dans lequel un hydrocarbure aromatique est utilisé en tant que solvant réactionnel.

4. Procédé de production selon la revendication 1, dans lequel du toluène est utilisé en tant que solvant réactionnel.

5. Procédé de production selon l'une quelconque des revendications 1 à 4, dans lequel la quantité de solvant réactionnel à utiliser est de 1 à 50 fois en masse celle du composé dinitro représenté par la formule 1.

6. Procédé de production selon l'une quelconque des revendications 1 à 5, dans lequel la réaction est effectuée à une température de 50 à 120°C.

7. Procédé de production selon l'une quelconque des revendications 1 à 6, dans lequel la réaction est effectuée sous une pression réduite, de 100 à 700 mm Hg en pression absolue.

8. Procédé de production selon l'une quelconque des revendications 1 à 7, dans lequel la quantité du composé représenté par la formule 2 à utiliser est de 0,5 à 3,0 fois en moles celle du composé dinitro représenté par la formule 1.

9. Procédé de production selon l'une quelconque des revendications 1 à 8, dans lequel le composé représenté par la formule 2 est un composé représenté par la formule 4.
